Europäisches Patentamt

European Patent Office

Office européen des brevets

(11) Publication number: **0 283 442 B1**

(12) **EUROPEAN PATENT SPECIFICATION**

(45) Date of publication of patent specification: **07.10.92**    (51) Int. Cl.5: **A61K 9/54**, A61K 37/54

(21) Application number: **88830018.3**

(22) Date of filing: **18.01.88**

(54) **Process for the manufacture of gastro-resistant and enterosoluble small spheres of digestive enzyme and pharmaceutical preparation so obtained.**

(30) Priority: **21.01.87 IT 4754687**

(43) Date of publication of application:
**21.09.88 Bulletin 88/38**

(45) Publication of the grant of the patent:
**07.10.92 Bulletin 92/41**

(84) Designated Contracting States:
**AT BE CH DE ES FR GB GR LI LU NL SE**

(56) References cited:
**EP-A- 0 021 129**
**DE-B- 1 617 363**

**P.H. LIST et al.: "Hagers Handbuch der Pharmazeutischen Praxis", edition 4, vol. 7, part A, 1971, page 313, Springer-Verlag, Berlin, DE**

(73) Proprietor: **SANDOZ S.A.**
**Lichtstrasse 35**
**CH-4002 Basel(CH)**

(72) Inventor: **D'Amico, Paolo**
**152 Viale Cortina d'Ampezzo**
**I-00135 Roma RM(IT)**

Rank Xerox (UK) Business Services

## Description

The present invention relates to a process for the manufacture of a pharmaceutical preparation in the form of very small gastro-resistant and enterosoluble spheres or microspheres for the administration of a digestive enzyme, particularly pancrelipase enzyme, as well as to a pharmaceutical preparation consisting of such microspheres, for an oral administration of said enzyme to patients in need thereof at the duodenal level.

Pharmaceutical preparations in the form of microspheres are known to the art, comprising a digestive enzyme. All these microspheres comprise an inner seed of irregular shape, covered by a layer which serves the purpose of sphering and uniformizing the microspheres, while at the same time enlarging their size to some extent, and an enteric outer coating which can withstand the acidic gastric environment and is dissolved, conversely, in the alkaline duodenum environment.

A known form of said microspheres comprises a substantially inert inner seed of extraneous nature (grain of sugar, ground wheat or the like) on which an enzyme layer is accumulated by sprinkling of a binding solution and dusting with enzyme powder. After obtaining the desired weight and size of the sphere, an enteric coating is applied thereon. In this prior art type of microsphere, however, the enzyme is present in the enlarging and spheration layer, in intimate contact with the binder and, during preparation, the solvent.

In U.S. patent number 4079125 small spheres or microspheres are described in which a seed granule is produced by extrusion from a paste consisting of an enzyme powder and a binding system comprising a binder, a stabiliser and a disintegrant. The granule so obtained is then directly coated with the protective enteric coating.

For the manufacture of this type of pharmaceutical preparation the starting materials to be used are well known.

While the invention could be applied to any type of enzyme powder able to form a coherent material when subjected to a compression operation, the process of the invention is particularly advantageous in the preparation of microspheres for the administration of pancrelipase or pancreatic enzymes which are available in the form of a concentrate powder. The binding solution is also known per se in its components. Particularly isopropylalcohol can be used as a solvent and polyvinylpyrrolidone can be used as a binder. Consequently, in the following, reference is made to the above substances, without excluding, however, the possibility of using other components in accordance with the teaching of the prior art.

A disadvantage in the microspheres of the prior art consists in that the enzyme, both during the microsphere preparation and within the microsphere itself, is in direct contact with other substances, such as the binder, the solvent, the disintegrant (particularly citric acid) and eventual preservants. This represents a draw-back in that the intimate contact with such substances could cause in time a degradation of the enzyme itself, which per se is a particularly active agent. It would be desirable, in fact, that the enzyme should not come into contact, either during the manufacturing process or in the preparation, with other substances.

Moreover it should be noted that it would be desirable that the size of the enzyme sphere be as small as possible, to be more easily dissolved at the duodenal level in order to release the enzyme as swiftly as possible. To render dissolution easier, the microsphere described in the above cited US patent number 4079125, contains in fact a disintegrant. The process of the present invention is able to form sufficiently small seed granules without any use of extraneous substances.

Furthermore, it would be desirable that in the manufacturing process there be the least number of rejects and losses possible, in view of the high cost of the enzyme. The process of the invention shows very reduced losses, in that the residue from sieving, as will be seen hereinafter, can be totally recovered.

In EP 21129 there is described a process for the production of pancreatin pellets wherein initially pancreatin powder , a solvent and additives, e.g. magnesium stearate or a hydrocarbon, is mixed, and the resultant paste is extruded to produce cylindrical cuttings , which are then rounded ( sphered ), and an outer enteric coating provided. This disclosure does not suggest the presently claimed invention which uses a dry process to form the inner pancreatin seed free of any additives.

Consequently, according to the present invention, a process for manufacturing microspheres to be used for the pharmaceutical administration of pancreatic digestive enzymes, in which the active principle is in the form of a powder of enzyme concentrate and which comprises the steps of

(a) preparing an inner seed of the microsphere,

(b) sphering the microsphere, and

(c) applying an outer enteric coating, is characterized in that for step (a), the enzyme concentrate powder is compressed into tablets and successively granulated under dry conditions to obtain granules with a

2

particle size form 0,5 to 1mm, to be used as inner seeds for the microspheres, said granules being made only of said enzyme concentrate; and in step (b) said granules are sprinkled with a binding solution and dusted with additional enzyme concentrate powder, while said granules are rolled to form all around said granules a substantially spherical layer made of the enzyme concentrate and the residue of the binding solution after evaporation of solvent.

The microspheres thus produced show an inner seed made exclusively of the enzyme concentrate under the form of compressed powder, whilst the intermediate spheration layer is made of a composition of said enzyme concentrate and the residue after solvent evaporation of a binding solution.

It will be noted that, according to the present invention, the granule which forms the seed is made of pure pancrelipase, without any addition of excipients and without resorting to spherical particles of extraneous nature.

This makes it possible to positively and almost totally recover any discarded residue of unacceptable size, making the final yields high. Moreover, no type of wet granulation, which could affect the enzyme stability, is necessary. According to the process of the invention, moreover, it is possible to reduce the size of the initial granule to an order of 0,75 ± 0,25 mm, making it possible, in view of its increased surface, to obtain a swifter and more reliable evaporation of the solvents during the enlarging step, an enteric coating of better quality, a more efficient release of enzymes in the intestinal tract and a lower error in the filling of the containing capsule.

The possibility of obtaining small granules derives from the fact that they are produced through milling of hard tablets and consequently they are harder and less friable than those obtained in the prior art by means of extrusion of a paste or simple accumulation on a seed of extraneous nature.

As the granules are hard per se, they are not liable to turn to powder during working, so that the rejects are reduced and uniformity is increased.

In view of the fact that the contact with the solvents, however "inert" they might be, is practically reduced to a minimum during both the enlarging-sphering step and the enteric coating step, results of tests of the enzyme activity on the finished microsphere have shown practically no decrease of enzyme titer.

The efficiency of the enteric uniform coating afforded by the microsphere geometry is confirmed by checking tests carried out on gastro-resistant granules: more than 95% of the microspheres remain unchanged following the simulated gastric juice test pun for a longer time than that required by the pharmaceutical standards.

As far as the enterosolubility is concerned, the increased surface obtained with the presence of a small seed granule, implies a widespread attack of the enteric liquid, which produces a complete release of the pancreatic enzymes and a more advantageous disposability thereof.

The present invention will be described in greater detail with reference to the following examples.

**Example of preparation - 1**

1. Starting material

The pancrelipase used in the preparation of microspheres by the process according to the invention, had the characteristics indicated in USP XX1 edition. The units of measure for the evaluation of the enzyme activity are also defined there.

A pancrelipase was used having, per mg:

| - lipase activity | 38 USP u. equal to 38 FU u. page 348 vol. I |
| - amilase activity | 125 USP u. equal to 30 FU u. page 348 vol. I |
| - protease activity | 100 USP u. equal to 1,6 FU u. page 348 vol. I |

2. Preparation of the granules

5 Kg pancrelipase were directly compressed by a compressing machine with no addition of any excipient, the raw material being easily compressible and any weight variation being of no interest. Punches of 20 mm size were used.

The tables so obtained were milled and passed on an Alexanderwerk type granulator through a 1 mm sieve.

Screening of the granules was finished by sieving once more on a 0,5 mm sieve. The residue on the 0,5 mm sieve was collected and the powder portion passing through the sieve was compressed and granulated again, sieved once more and the residue on 0,5 sieve was collected and united to the previously collected portion. The operation was repeated to transform, by successive enrichments, the whole initial pancrelipase into a granulate of uniform granule diameter and particle size not higher than 1 mm and not lower than 0,5 mm.

## 3. Sphering

The granules obtained according to the foregoing described method, were transferred into a revolving steel pan having a geometry different from that of a conventional revolving pan. In fact, this revolving pan had a barrel configuration with a raised frusto-conical rim having, in addition to the conventional rotation ability, the possibility of inclining its axis to a horizontal position. The barrel should moreover be provided with an aspirator and blower for controlling the temperature of warm air. The barrel permits an easy rolling of the granules. After the pan having begun to rotate, a 7% solution of polyvinylpyrrolidone in isopropylalcohol (binding solution) was sprayed onto the rolling granules, care being taken to wet only the rolling mass, but not the pan walls.

An amount of about 120 to 150 g solution was dispensed.

The operation was repeated for an additional five times before the granules were completely dried.

Thereafter the granules were coated by pancrelipase powder, again of the initial type 30 FU.

To this end 100 g pancrelipase were dispensed onto the dried granules, accurately stirring the mass having a high particle size differential in order to obtain the most homogeneous distribution possible, and the mass was wetted with 150 g binding solution, taking the aforementioned precautions.

In this step it is advantageous to stir the rotating mass vigorously, taking care that the granules are sufficiently wet and avoiding the formation of granule agglomerates as far as possible. In the event of this occurring, it is necessary to add more pancrelipase powder under vigorous stirring until the granule agglomerates are completely loosened.

The entire operation described above was repeated a number of times until a total of 200 g pancrelipase powder had been absorbed. Evaporation of solvent should be aided by means of a light current of air. Moreover an adequate aspiration would protect the operator from isopropanol vapour in addition to aiding the evaporation.

During the successive and repeated steps of enlargement and sphering, the inclination of the pan axis should remain as far as possible horizontal, as it is in this position that the granules, on springing, tend to assume a spherical form. Of course the inclination of the axis can be modified according to the requirements of the various steps of enlargement.

After distributing the desired pancrelipase powder, the pan was discharged and the spheres were collected on a tray having a perforated bottom and brought to a drying oven for two hours at a temperature of 40°C.

Of the microspheres so obtained those passing through a 1,4 mm sieve, but not through a 1,25 mm sieve were selected.

The microspheres passing through the 1,25 mm sieve were returned to a revolving pan and further enlarged and sphered for a fresh dusting with pancrelipase powder.

The selection was continued by carrying out further collections, until a minimum amount of granules remained, such that it was no longer possible to obtain a satisfactory rotation.

The residue, consisting of granules which could no longer be processed, was recovered for its raw material content in the succeeding operation.

In any case a yield higher than 90% was obtained.

## 4. Coating of the microspheres with gastro-resistant varnish

Coating of the microsphere was carried out according to an established conventional method for giving tablets or other granules a protection against gastric conditions. To this purpose, cellulose acetophthalate was employed, for a polymer of which a wide positive experimentation is known, the gastro-protective efficiency of which on pancreatic enzymes is also known to those skilled in the art.

The coating solution was prepared according to the following composition:

| | |
|---|---|
| Cellulose acetophthalate | 120 g |
| Diethyl-phthalate | 30 g |
| Acetone | 500 ml |
| Methylene chloride to | 1000 ml |

The presence of diethylphthalate provides the coating solution with improved plastifying characteristics.

The coating solution was diluted 2:1 before being put into the process together with a mixture 1:1 of acetone and methylene chloride and repeatedly dispensed with the same method as above, so as to have finally 60 g cellulose acetophthalate for each initial Kg of microspheres.

During dispensing of the gastro-resistant varnish it is advantageous to dust the revolving microsphere mass with small amounts of magnesium stearate which aids in decreasing aggregation.

In this working stage also it is advisable to aid the solvent evaporation by a current of warm air and moderate application.

The final bulk density of the microspheres so obtained, is about 0,7 g/ml.

**Example of preparation - 2**

1. Preparation of the granules

The granules were prepared by preliminary compression of 5,5 NF(38 FIP) pancreatin.

The preparation was carried out by compressing pancreatin directly in a Ronchi type rotating machine. The tablets so obtained were granulated and passed again in the compressing machine. The pressure was set at 1000 Kg/cm$^2$ to obtain tablets as hard and strong as possible.

The granules were obtained by passing the tablets previously milled on an Alexanderwerk, through a 1,25 mm sieve. The finer portion of the granulate was removed by sieving through a 1 mm sieve.

1,25 Kg granulate were placed into a cylindrical revolving pan and revolved for about 2 hours at a mean speed. As a consequence of this operation, the granules were blunted and the resulting granulate was more uniform.

2. Preparation of the solutions

| Solution A (binding solution) | |
|---|---|
| Polyvynylpyrrolidone | 0,100 Kg |
| Isopropyl alcohol | 0,900 liters |
| Suspension B | |
| Solution A | 0,850 liters |
| Pancreatin 38 FIP | 0,150 Kg |
| Solution C | |
| Ethyl alcohol | 0.800 liters |
| Shellak | 0,200 Kg |
| Solution D (gastro-resistant varnish) | |
| Acetone | 0,500 liters |
| Methylene chloride | 0,500 liters |
| Cellulose acetophthalate | 0,100 liters |

3. Preparation of the microspheres

After starting the revolving pan, the granules were wetted with 60 to 70 ml of solution C. The granules were left to dry and wetted again with the same amount of solution. This operation was repeated five times.

The granules were brought into a warm air oven and dried to a temperature of 38 to 40°C for about one hour.

The granules were charged into the pan again and wetted initially with 50 ml of suspension B. This operation was repeated five times, taking care to be sure that the material in the pan was dry between one operation and the other, but not too dry, in that this could lead to formation of pancreatin powder. Drying with air aspiration is recommended during these operations.

The formation of the microspheres was carried out initially by wetting with suspension B and dusting with 38 FIP pancreatin powder. Each time the material is treated with suspension B, 20 to 30 g pancreatin should be added. Such amounts can be increased or decreased, according to the conditions in which the granulate is found. The process was continued until the material had absorbed about 400 g of powder.

At this stage round microspheres of pancreatin were formed.

It is required that all the microspheres pass through a sieve n. 12 and do not remain on a sieve n. 20.

### 4. Coating of the microspheres with gastro-resistant varnish

The microspheres were brought into a dryer and left at 30 to 40°C for about 4 hours. Then they were placed in the revolving pan again and treated with solution C 10 times, letting the microspheres be well dried after each application by means of air aspiration. The microspheres were placed in an oven at 30-40°C for about 1 hour, then placed into the pan again and the solution D was applied in a number of small doses by means of a sprayer. After about 1 liter of solution had been dispensed, the microspheres were sent to be checked for protection against gastric conditions.

5. Analysis of results

| Material used | | Microspheres obtained |
|---|---|---|
| Granulate | Kg 1,250 | |
| Pancrelipase powder | Kg 0,800 | |
| Shellak | Kg 0,030 | |
| P.V.P. | Kg 0,220 | |
| Talc | Kg 0,050 | |
| C.A.P. | Kg 0,070 | |
| | Kg 2,420 | Kg 2,340 |
| Eccipients | 15,3 % | |
| Yield | 96,6 % | |

ANALYTICAL DATA

Expressed as U FIP/mg and U USP/mg

| Initial enzymes | | Enzymes in microspheres | |
|---|---|---|---|
| 38 U FIP - 38 U USP | LIPASE | 31 U FIP - 31 U USP | |
| 31 U FIP - 128 U USP | AMYLASE | 25 U FIP - 104 U USP | |
| 1,65 U FIP - 103 U USP | PROTEASE | 1,4 U FIP - 88 U USP | |

The above described preparation is suited to be exploited on a commercial scale in the pharmaceutical industry.

While the invention has been described in its general aspect and in detailed examples, it will be obvious to those skilled in the art that alternative embodiments can be envisaged within its scope.

**Claims**

1. A process for manufacture of microspheres to be used for the pharmaceutical administration of pancreatic digestive enzymes, in which the active principle is in the form of a powder of enzyme concentrate, comprising the steps of
   (a) preparing an inner seed of the microsphere,
   (b) sphering the microsphere, and
   (c) applying an outer enteric coating,
   characterized in that:
      for the step (a) said enzyme concentrate powder is compressed into tablets, in a per se known manner, and successively dry granulated to obtain granules with a particle size from 0,5 to 1 mm to be used as inner seeds for the microspheres, said granules consisting only of said enzyme concentrate; and for step (b) said granules are sprinkled with a per se known binding solution and dusted with additional enzyme concentrate powder, while said granules are rolling, to form all round said granules a substantially spherical layer consisting of said enzyme concentrate and the residue of the binding solution after evaporation of solvent.

2. A process according to claim 1, in which said dry granulation is carried out by milling said tablets and sieving the granulate so obtained.

3. A process according to claim 2, in which the granulate material discarded in said sieving operation, is compressed again and recycled to the dry granulating operation.

4. A process according to claim 1, in which said binding solution comprises polyvinylpirrolidone and isoprophyl alcohol.

5. A process according to claim 4, in which the concentration of polyvinylpyrrolidone is 7%.

6. A pharmaceutical preparation for oral administration of pancreatic digestive enzymes, in the form of microspheres comprising an enterosoluble inner seed, an intermediate sphering layer and an outer enteric coating, characterized in that said inner seed in the microspheres consists solely of a concentrate of said enzyme in the form of compressed powder prepared according to claim 1 and said intermediate sphereing layer consists of a per se known composition of (a) said enzyme concentrate and (b) the residue of a binding solution after solvent evaporation.

7. Preparation according to claim 6, in which said seeds are of a particle size from 1 to 0,5 mm.

**Patentansprüche**

1. Ein verfahren zur Herstellung von Mikrokugeln für die Verwendung zur pharmazeutischen Verabreichung von Pankreas-Verdaungsenzymen worin sich der Wirkstroff in Form eines pulverförmigen Enzymkonzentrats befindet umfasst die Stufen
a.) Herstellung eines inneren Keims der Mikrokugel,
b.) Gestaltung der Mikrokugel und
c.) Anbringen einer äusseren darmlöslichen Schicht dadurch gekennzeichnet, dass für die Stuffe a.) das pulverförmige Enzymkonzentrat auf an sich bekannte Weise in Tabletten verpresst und sukzesive trockengranuliert wird um Granulate mit einer Teilchengrösse von 0,5 bis 1 mm zu erhalten, die als innerer Keim der Mikrokugeln verwendet werden, wobei diese Granulate nur aus dem Enzymkonzentrat bestehen; und für die Stufe b.) diese Granulate mit einer an sich bekannten, als Bindemittel dienenden, Lösung besprüht und mit weiterem pulverförmigem Pulverkonzentrat bestäubt werden, währenddessen diese Granulate so gestaltet werden, dass man eine im wesentlichen kugelförmige Schicht erhält die aus dem besprochenen Enzymkonzentrat und dem Rückstand der als Bindemittel verwendeten Lösung nach Verdampfen des Lösungsmittels besteht.

2. Ein verfahren gemäss Anspruch 1.) worin die Trockengranulierung durch Vermahlen der Tabletten und Sieben der so erhaltenen Granulate erfolgt.

3. Ein Verfahren gemäss Anspruch 2.) worin das bei der Siebeoperation verworfene Granuliermaterial neuerlich verpresst und dem Trockengranulierungsvorgang zugeführt wird.

4. Ein Verfahren gemäss Anspruch 1.) worin die als Bindemittel verwendete Lösung Polyvinylpyrrolidon und Isopropylalkohol enthält.

5. Ein Verfahren gemäss Anspruch 4.) worin die Konzentration von Polyvinylpyrrolidon 7% beträgt.

6. Eine pharmazeutische Zubereitung zur oralen Verabreichung von Pankreas-Verdaungsenzymen in Form von Mikrokugeln enthaltend einen dünndarmlöslichen inneren Keim, eine kugelförmige Zwischenschicht und eine äussere darmlösliche Schicht dadurch gekennzeichnet, dass der innere Keim der Mikrokugeln ausschliesslich aus einem Konzentrat des Enzyms in Form eines verpressten Pulvers, das gemäss Anspruch 1.) hergestellte wurde, besteht und die kugelförmige Zwischenschicht aus einer an sich

EP 0 283 442 B1

bekannten Zusammensetzung enthaltend a.) das besprochene Enzymkonzentrat und b.) den Rückstand der als Bindemittel verwendeten Lösung nach Verdampfen des Lösungsmittels besteht.

7. Zubereitung gemäss Anspruch 6.) worin die Keime eine Teilchengrösse von 1 bis 0,5 mm besitzen.

**Revendications**

1. Un procédé de préparation de microsphères à utiliser pour l'administration pharmaceutique d'enzymes digestives pancréatiques, dans lesquelles le principe actif est sous forme d'une poudre du concentré d'enzyme, comprenant les étapes de
   a) préparation d'un noyau interne de la microsphère,
   b) sphéronisation de la microsphère et
   c) application d'un revêtement externe gastro-résistant,
   caractérisé en ce que :
   pour l'étape a) ladite poudre du concentré d'enzyme est comprimée d'une manière connue en soi en comprimés et successivement granulée à sec pour obtenir des granules d'une granulométrie comprise entre 0,5 et 1 mm à utiliser comme noyaux internes pour les microsphères, lesdites granules étant constituées uniquement dudit concentré d'enzyme; et pour l'étape b) lesdites granules sont pulvérisées avec une solution liante connue en soi et saupoudrées avec de la poudre supplémentaire du concentré d'enzyme, pendant que lesdites granules sont en rotation, pour former autour desdites granules une couche substantiellement sphérique constituée dudit concentré d'enzyme et du résidu de la solution liante après évaporation du solvant.

2. Un procédé selon la revendication 1, dans lequel ladite granulation à sec est effectuée par broyage desdits comprimés et tamisage du granulé ainsi obtenu.

3. Un procédé selon la revendication 2, dans lequel la matière du granulé écartée dans ladite opération de tamisage, est comprimée à nouveau et recyclée à l'opération de granulation à sec.

4. Un procédé selon la revendication 1, dans lequel ladite solution liante comprend de la polyvinylpyrrolidone et de l'alcool isopropylique.

5. Un procédé selon la revendication 4, dans lequel la concentration de polyvinylpyrrolidone est de 7%.

6. Une préparation pharmaceutique pour l'administration par voie orale d'enzymes digestives pancréatiques, sous forme de microsphères comprenant un noyau interne entéro-soluble, une couche intermédiaire de sphéronisation et un revêtement externe gastro-résistant, caractérisée en ce que ledit noyau interne dans les microsphères est constitué uniquement d'un concentré de ladite enzyme sous forme de poudre comprimée préparée selon la revendication 1 et ladite couche intermédiaire de sphéronisation est constituée d'une composition connue en soi de a) ledit concentré d'enzyme et b) le résidu après évaporation du solvant d'une solution liante.

7. Préparation selon la revendication 6, dans laquelle lesdits noyaux ont une granulométrie comprise entre 1 et 0,5 mm.

9